# EUROPÄISCHE PATENTANMELDUNG

(11) **EP 2 106 783 A1**
(43) Veröffentlichungstag der Anmeldung: **07.10.2009**
(21) Anmeldenummer: 09150711.1
(22) Anmeldetag: 16.01.2009
(51) Int. Cl.: A61K 8/35, A61K 8/37, A61K 8/49, A61Q 17/04

(54) **Sonnenschutzmittel**

(30) Priorität: 31.03.2008 DE 102008016709
(71) Anmelder: Sebapharma GmbH & Co. KG, 56154 Boppard-Bad Salzig (DE)
(72) Erfinder: Gottfreund, Joachim, 56154, Boppard (DE); Meyer, Thomas, 56281, Emmelshausen (DE)
(74) Vertreter: Lippert, Hans-Joachim

(57) **Zusammenfassung**

Die Erfindung betrifft ein Sonnenschutzmittel enthaltend einen kosmetisch oder dermatologisch akzeptablen Träger und als UV-Filter
(i) ein Crylen
(ii) ein Benzoylmethan und/der Imidazol und
(iii) ein Triazin und/oder ein Triazon,

wobei das Sonnenschutzmittel einen pH-Wert von 3,5 bis 5,5 und einen Lichtschutzfaktor SPF von ≥ 30 aufweist. Weiterhin sind vorzugsweise Inulin, Dextran, Dextransulfat einzeln oder in Kombination enthalten.

## Beschreibung

Die Erfindung betrifft ein Sonnenschutzmittel enthaltend einen kosmetisch oder dermatologisch akzeptablen Träger und einen UV-Filter, wobei das Sonnenschutzmittel einen Lichtschutzfaktor SPF von ≥ 30 aufweist.

Derartige Sonnenschutzmittel sind in unterschiedlichen Anwendungsformen wie z. B. als Öl, Milch, Creme, Spray oder dergleichen mit einer Vielzahl unterschiedlicher Komponenten als UV-Filter bekannt. Die UV-Filter-Komponenten soll zum Einen sowohl UV-A Licht als auch UV-B Licht möglichst vollständig absorbieren, um eine Schädigung der Haut sowohl im Hinblick auf Hautrötungen als auch Entstehung genetischer Defekte durch UV-B Strahlung zu verhindern. Weiterhin sollen die UV-Filter-Komponenten in dem jeweiligen Träger nach Möglichkeit in gelöster Form vorliegen, um so die Wirksamkeit der Filterkomponenten zu erhöhen und die Anwendung des Sonnenschutzmittels zu erleichtern. Andererseits bedingt der Einsatz gelöster UV-Filter-Komponenten Probleme bezüglich der Stabilität der Sonnenschutzmittel, die bei unterschiedlichen Temperaturen einsetzbar sein müssen, beispielsweise sowohl bei sommerlichen Temperaturen als auch bei winterlichen Temperaturen z.B. bei Verwendung der Sonnenschutzmittel im Gebirge. Insbesondere sind auch hohe Anforderungen an die Langzeitlagerbeständigkeit des Sonnenschutzmittels zu stellen.

Diese Anforderungen, die von herkömmlichen Sonnenschutzmitteln in der Regel in ausreichendem Maße erfüllt werden, werden jedoch weiter verschärft, wenn die Sonnenschutzmittel auch sehr hohe Lichtschutzfaktoren von z. B. ≥ 30 aufweisen sollen und zugleich niedrige pH-Werte von ≤ 5,5. So erfordern hohe Lichtschutzfaktoren vergleichsweise hohe Konzentrationen der UV-Filter-Komponenten. Andererseits führen niedrige pH-Werte von ≤ 5,5 zu einer verstärkten Kristallisation der UV-Filter-Komponenten, wodurch zum Einen die Wirksamkeit der Sonnenschutzmittel vermindert und zum Anderen deren Anwendungseigenschaften unerwünscht verändert werden. Ein niedriger pH-Wert von ≤ 5,5 hat sich jedoch als vorteilhaft erwiesen, da er die Stabilität der Haut fördert, insbesondere bei starker Sonnenbestrahlung. Andererseits bedingt ein niedriger pH-Wert insbesondere in Kombination mit einem hohen Lichtschutzfaktor besondere Probleme bezüglich der Stabilität des Sonnenschutzmittels, da dann die UV-Filter-Komponenten verstärkt zum Ausflocken bzw. Auskristallisieren neigen. Ein Sonnenschutzmittel, welches auch bei einem hohen Lichtschutzfaktor SPF von ≥ 30 und einem niedrigen pH-Wert von ≤ 5,5 eine ausreichende Langzeitstabilität aufweist, insbesondere auch bei niedrigen Temperaturen, ist noch nicht beschrieben worden.

Der Erfindung liegt daher die Aufgabe zugrunde, ein Sonnenschutzmittel bereitzustellen, welches bei hohen Lichtschutzfaktoren von ≥ 30 und bei niedrigen pH-Werten von ≤ 5,5 eine hohe Langzeitstabilität auch bei tiefen Temperaturen aufweist.

Überraschenderweise kann die Aufgabe durch ein Sonnenschutzmittel nach Anspruch 1 gelöst werden, bei welchem eine Kombination der UV-Filter-Komponenten bestehend aus (i) einem Crylen, (ii) mindestens einem Benzoylmethan und/oder mindestens einem Imidazol und (iii) mindestens einem Triazin und/oder mindestens einem Triazon vorliegt. Ein Sonnenschutzmittel, in welchem diese UV-Filter-Komponenten in Kombination vorliegen, weist auch bei hohen Lichtschutzfaktoren von ≥ 30 und niedrigen pH-Werten von ≤ 5,5 eine sehr gute UV-Filter-Wirkung sowohl im UV-A als auch im UV-B-Bereich und zugleich eine sehr hohe Langzeitlagerstabilität auch bei tiefen Temperaturen auf. Trotz des sehr niedrigen pH-Wertes besteht praktisch keine Tendenz zur Auskristallisation oder Ausflockung der UV-Filter-Komponenten. Zwar ist die Verwendung der oben genannten Verbindungen als UV-Filter-Komponenten als solche bereits bekannt, überraschenderweise kann jedoch durch die Auswahl dieser Komponenten aus der überaus großen Vielzahl an sich bekannter UV-Filter-Komponenten ein UV-Filtersystem geschaffen werden, welches auch bei sehr niedrigen pH-Werten und hohen Gehalten der Filterkomponenten zur Bereitstellung hoher Lichtschutzfaktoren eine sehr große Stabilität und praktisch keine Neigung zur Auskristallisation aufweist. Dies überrascht umso mehr, als dass der Bedarf an der Bereitstellung eines Sonnenschutzmittels mit niedrigem pH-Wert und hohem Lichtschutzfaktor bereits seit langem besteht, ohne unter Berücksichtigung der weiteren Anforderungen zufriedenstellend gelöst zu werden.

In dem erfindungsgemäßen Sonnenschutzmittel kann insbesondere mindestens ein Benzoylmethan in Kombination mit mindestens einem Triazin und/oder mindestens einem Triazon vorliegen. Für bestimmte Anwendungsfälle hat es sich jedoch auch als besonders vorteilhaft erwiesen, wenn mindestens ein Imidazol, insbesondere ein Benzimidazolsalz, in Kombination mit mindestens einem Triazin und/oder mindestens einem Triazon vorliegt, wobei jeweils zugleich ein Crylen enthalten ist, vorzugsweise jeweils Octocrylen. Gegebenenfalls können auch die vier genannten Komponenten gleichzeitig in Kombination mit einem Crylen vorliegen, vorzugsweise jeweils Octocrylen.

Besonders bevorzugt enthält das Sonnenschutzmittel mindestens einen oder mehrere der Stoffe ausgewählt aus der Gruppe Inulin, Dextran, Dextransulfat. Es hat sich herausgestellt, dass bei Anwesenheit mindestens einer oder mehrerer dieser Stoffe die Stabilität des Sonnenschutzmittels bei niedrigen pH-Werten und hohen Lichtschutzfaktoren besonders wirksam weiter erhöht werden kann. Insbesondere kann Inulin in Kombination mit Dextran und/oder Dextransulfat vorliegen, gegebenenfalls auch nur eine Kombination von Dextran und Dextransulfat oder nur einer der genannten Verbindungen oder die drei genannten Verbindungen in Kombination.

Inulin (auch Alantstärke genannt), welches ein Polysaccharid aus Fruktosemolekülen und einem endständigen Glukoserest ist, kann in niedermolekularer Form eingesetzt werden, beispielsweise mit 10 bis 50 oder 20 bis 30 Fruktosemolekülen, gegebenenfalls auch in hochmolekularer Form mit Kettenlängen von 50 bis 100 Molekülen oder mehr, beispielsweise 50 bis 70 oder 70 bis 100 Molekülen, gegebenenfalls auch in Kettenlängen von 20 bis 70 oder 30 bis 50.

Als Dextrane können insbesondere solche eingesetzt werden, die von Bakterien der Gattung Leuconostoc gebildet werden, beispielsweise von L.mesenteroides und/oder L.dextranicum. Eingesetzt werden können insbesondere niedermolekulare Dextrane mit einem Molgewicht von ca. 1,5 x 10⁴ bis 1 x 10⁵ oder bis 1 x 10⁶, gegebenenfalls auch solche mit einem Molekulargewicht von bis zu 1 x 10⁷ oder bis 5 x 10⁷. Gegebenenfalls können auch hochmolekulare Dextrane mit einem Molgewicht von ca. 1 x 10⁶ bis 5 x 10⁷ eingesetzt werden.

Die eingesetzten Dextransulfate als Halbester des Dextrans können 1 bis 2 Sulfatgruppen pro Anhydroglukosebaustein aufweisen. Vorzugsweise werden die Dextransulfate als Alkalisalze, insbesondere Natriumsalze eingesetzt.

Inulin, Dextran und/oder Dextransulfat können jeweils einzeln oder in Kombination in einem Gehalt von ≥ 0,01 Gew.-% enthalten sein, beispielsweise in einem Gehalt von 0,01 bis 1 Gew.-% oder auch bis 2 Gew.-%, insbesondere 0,02 bis 0,5 Gew.-% oder 0,025 bis 0,2 Gew.-%, besonders bevorzugt 0,05 bis 0,2 Gew.-% oder ca. 0,1 Gew.-%.

Zur Erhöhung der Stabilität des Sonnenschutzmittels mit niedrigen pH-Werten und tieferen Temperaturen hat es sich als besonders vorteilhaft herausgestellt, wenn weiterhin Lecithin enthalten ist, beispielsweise in einem Gehalt von 0,01 bis 1 oder bis 2 Gew.-%, insbesondere 0,02 bis 0,5 Gew.-% oder 0,025 bis 0,2 Gew.-%, besonders bevorzugt 0,05 bis 0,2 Gew.-%. Insbesondere kann Lecithin in Kombination mit den Stoffen Inulin und/oder Dextran oder Dextran und/oder Dextransulfat vorliegen, gegebenenfalls auch in der Kombination Inulin und Dextransulfat oder in Kombination mit den drei genannten Komponenten.

Als Crylen seien hier Zimtsäureester verstanden, insbesondere EMI6.3 Ester der Zimtsäure, vorzugsweise Methoxyzimtsäureester wie 4-Methoxyzimtsäaure-2-ethylhexylester, 4-Methoxyzimtsäaurepropylester, beispielsweise Zimtsäureisoamylester wie 4-Methoxyzimtsäureisoamylester, besonders bevorzugt Phenylzimtsäureester, einschließlich Cyanophenylzimtsäureester wie 2-Cyano-3,3-phenylzimtsäure-2-ethylhexylester (Octocrylene). Die Ester sind bevorzugt solche mit C2-C14 Alkoholen, insbesondere Alkanolen, beispielsweise mit C4-C12- oder C4-C10-Alkoholen oder C4-C8-Alkoholen.

Bevorzugt weist das Sonnenschutzmittel als Crylen Octocrylen auf. Das Crylen, insbesondere Octocrylen, kann in einem Gehalt von ≥ 1 bis 2 Gew.-% bezogen auf das Gesamtgewicht des Sonnenschutzmittels vorliegen, beispielsweise von 1 bis 20 Gew.-%, insbesondere 2 bis 15 Gew.-%, vorzugsweise 4 bis 12 Gew.-% oder 6 bis 10 Gew.-% bezogen auf das Gesamtgewicht des Sonnenschutzmittels.

Vorzugsweise liegt als Benzoylmethan mindestens ein Dibenzoylmethan vor, besonders bevorzugt ein Alkylalkoxydibenzoylmethan, wobei Alkyl 1 bis 12 C-Atome, 2 bis 8 oder 2 bis 8 C-Atome, besonders bevorzugt 3 bis 5 C-Atome oder meist bevorzugt 4 C-Atome aufweist. Die Alkoxygruppe kann 1 bis 5 C-Atome oder 1 bis 4 C-Atome aufweisen, besonders bevorzugt 1 bis 2 oder 1 bis 3 C-Atome, meist bevorzugt liegt eine Methoxygruppe vor. Die Alkyl- und/oder Alkoxygruppen können jeweils linear oder verzweigt sein. Besonders bevorzugt wird Buthylmethoxydibenzoylmethan eingesetzt.

Das mindestens eine oder mehrere Benzoylmethane können in einem Gehalt von 0,1 bis 15 Gew.-% vorliegen, beispielsweise 0,2 bis 10 Gew.-% oder 0,5 bis 5 Gew.-%, insbesondere im Bereich von 1 bis 4 Gew.-%. Insbesondere kann das Benzoylmethan oder die Benzoylmethane Alkyl- und/oder Alkoxy-substituiert sein.

Durch die Kombination der gewählten UV-Filter-Komponenten bei niedrigem pH-Wert kann überraschenderweise auch bei vergleichsweise hohen Gehalten des Dibenzoylmethans eine relativ hohe Fotostabilität desselben erzielt werden, so dass die ansonsten begrenzte Fotostabilität der Dibenzoylmethanderivate bei der Anwendung auf der Haut verbessert werden kann.

Als Triazin kann mindestens ein Alkoxyphenyltriazin eingesetzt werden, welches Alkyl- und/oder Alkoxy-substituiert sein kann, wobei die Alkoxygruppe beispielsweise 1 bis 6 C-Atome, 1 bis 4 C-Atome oder ein, zwei oder drei C-Atome aufweisen kann, besonders bevorzugt Methoxyphenyltriazine. Die Alkoxygruppe kann linear oder verzweigt sein. Besonders bevorzugt sind Phenolalkoxyphenyltriazine bzw. Oxyphenolalkoxyphenyltriazine, insbesondere Bis-Alkylalkyloxyphenolalkoxyphenyltriazine, wobei die Alkylgruppe des Alkyloxyphenol-Restes 1 bis 8 C-Atome, vorzugsweise 1 bis 6 oder 2 bis 4 C-Atome aufweisen kann, bevorzugt 2 oder 3 C-Atome. Die Alkyloxygruppe des Alkyloxyphenolrestes kann 2 bis 12 C-Atome, vorzugsweise 4 bis 8 C-Atome oder 6 C-Atome aufweisen. Besonders bevorzugt ist Bis-Ethylhexyloxyphenolmethoxyphenyltriazin enthalten.

Eines oder mehrere der Triazine können in einem Gehalt bzw. Gesamtgehalt von ≥ 0,1 Gew.-% enthalten sein, vorzugsweise in einem Gehalt von 0,1 bis 10 Gew.-%, vorzugsweise 0,2 bis 5 Gew.-%, besonders bevorzugt 0,25 bis 4 Gew.-% oder 0,5 bis 2 Gew.-%, insbesondere ca. 1 Gew.-%, jeweils bezogen auf das Gesamtgewicht des Sonnenschutzmittels.

Vorzugsweise ist das in dem Sonnenschutzmittel enthaltene Triazon mindestens ein Amidotriazon, insbesondere ein Alkyl substituiertes Alkylamidotriazon, wobei neben der Alkylamidogruppe 1 bis 6, vorzugsweise 2 bis 4 oder mehr, beispielsweise 3 Alkylgruppen vorliegen können, die jeweils unabhängig voneinander 1 bis 10 C-Atome, vorzugsweise 2 bis 8 C-Atome, insbesondere 2 bis 6 C-Atome aufweisen können. Die Alkamidogruppe kann 2 bis 8 C-Atome aufweisen, insbesondere 3 bis 6 C-Atome oder 4 C-Atome. Besonders bevorzugt wird als Triazon Diethylhexylbutamidotriazon eingesetzt.

Unter Umständen sind auch Alkyltriazone bzw. Dialkyltriazone einsetzbar, beispeilsweise solche mit C1 bis C12- oder C1 bis C10-Alkylreste, insbesondere mit C1 bis C8-Alkylresten, wobei die Alkylreste voneinander verschieden sein können. Beispielhaft genannt sei Ethylhexyltriazon.

Das mindestens ein oder mehrere Triazone in dem Sonnenschutzmittel können in einem Gehalt bzw. Gesamtgehalt von ≥ 0,1 Gew.-% bezogen auf das Gesamtgewicht des Sonnenschutzmittels enthalten sein, vorzugsweise in einem Gehalt von 0,1 bis 10 Gew.-%, vorzugsweise 0,2 bis 5 Gew.-% oder 0,25 bis 4 Gew.-%, vorzugsweise 0,5 bis 4 Gew.-% oder im Bereich von 0,5 bis 2 Gew.-%.

Vorzugsweise ist das in dem Sonnenschutzmittel enthaltene Imidazol ein Benzylimidazol- oder Phenylbenzylimidazolderivat, insbesondere jeweils ein Salz desselben, wobei das Imidazol Alkyl- und/oder Alkoxy-substituiert sein kann. Die anionische Gruppe kann eine Sulfonatgruppe sein. Insbesondere kann ein Alkalimetallsalz oder Salz einer organischen Base, insbesondere ein Ammoniumsalz vorliegen, besonders bevorzugt ein Natriumsalz. Das Imidazol kann Alkyl-und/oder Alkoxy-substituiert sein, wobei der Alkyl- und/oder Alkoxyrest jeweils unabhängig voneinander 1 bis 6, 1 bis 4 oder 1 oder 2 C-Atome aufweisen kann, vorzugsweise liegen keine Alkyl- und/oder Alkoxy-Substituenten vor.

Das mindestens eine oder mehrere Imidazole können in einem Gehalt bzw. Gesamtgehalt von ≥ 0,1 Gew.-% bezogen auf das Gesamtgewicht des Sonnenschutzmittels enthalten sein, vorzugsweise in einem Gehalt von 0,1 bis 10 Gew.-%, beispielsweise 0,2 bis 5 Gew.-% oder 0,25 bis 4 Gew.-%, beispielsweise zwischen 0,5 und 2 Gew.-%.

Das Gewichtsverhältnis der Komponenten Benzoylmethan : Imidazol, Imidazol : Triazon und/oder Benzoylmethan : Triazon kann jeweils unabhängig voneinander 1:6 bis 6:1 oder 1:4 bis 4:1, vorzugsweise 1:3 bis 3:1 oder insbesondere 1:2 bis 2:1 betragen, besonders bevorzugt 1:1,5 bis 1,5: 1 oder ca. 1:1. Entsprechendes kann jeweils für das Verhältnis Imidazol : Triazin und/oder Benzoylmethan : Triazin gelten.

Gegebenenfalls kann das Gewichtsverhältnis der Komponenten Benzoylmethan : Triazin und/oder Benzoylmethan : Triazon jeweils unabhängig voneinander auch im Bereich von 12:1 bis 1:2 oder 10:1 bis 1:1, vorzugsweise 8:1 bis 2:1 oder insbesondere 6:1 bis 4:1 betragen.

Das Gewichtsverhältnis der Komponenten Triazin : Triazon kann jeweils 1:6 bis 6:1 oder 1:4 bis 4:1, vorzugsweise 1:3 bis 3:1 oder insbesondere 1:2 bis 2:1 betragen, besonders bevorzugt 1:1,5 bis 1,5: 1 oder ca. 1:1 betragen.

Das Gewichtsverhältnis der Komponenten Crylen : Benzoylmethan und/oder Crylen : Imidazol und/oder Crylen: Triazin und/oder Crylen: Triazon kann jeweils unabhängig voneinander 20:1 bis 1:1, vorzugsweise 16:1 bis 2:1 oder insbesondere 12:1 bis 3:1 betragen, besonders bevorzugt 10:1 bis 3: 1 oder ca. 4:1.

Gegebenenfalls kann das Gewichtsverhältnis der Komponenten Crylen : Benzoylmethan auch im Bereich von 10:1 bis 1:2 oder 8:1 bis 1:1, vorzugsweise 4:1 bis 1:1 oder insbesondere ca. 2:1 betragen.

Der pH-Wert des Sonnenschutzmittels liegt vorzugsweise in dem Bereich von 4 bis 5,5 oder zwischen 4,5 und 5,5. Bei einem derartigen pH-Wert kann die zu behandelnde Haut jeweils in besonderem Maße stabilisiert werden, gleichzeitig werden allzu niedrige pH-Werte, die Hautirritationen bewirken oder zu einer verstärkten Kristallisationsneigung der UV-Filter-Komponenten führen könnten, vermieden. Zudem wird hierdurch die Stabilität des Sonnenschutzmittels auf der Haut verbessert. Der pH-Wert kann insbesondere durch organische Säuren, besonders bevorzugt Carbonsäuren bzw. Hydroxycarbonsäuren wie Milchsäure, Weinsäure, Apfelsäure, Citronensäure oder deren Derivate eingestellt werden.

Das erfindungsgemäße Sonnenschutzmittel kann gegebenenfalls weitere organische UV-Filter-Komponenten aufweisen, beispielsweise eines oder mehrere aus der Gruppe der Anthralinate, Salizylate, Derivate des Kampfers, Derivate von Benzalmalonaten, Bis-Benzoacolyl, Derivate von Beta, Beta-Diphenylacrylaten, Silikonfilter, Derivate von Alpha-Alkylstyrenen, polymere Filter, gegebnenfalls auch Cinnamate, oder dergleichen. Weitere UV-Filterkomponenten können jedoch auch entbehrlich sein.

Weiterhin können Sonnenschutzmittelpigmente enthalten sein, insbesondere auch Nanopigmente oder Mikropigmente, die einen durchschnittlichen Durchmesser von 1 bis 150 Nanometer, beispielsweise 10 bis 100 oder 10 bis 50 Nanometer aufweisen können. Derartige Pigmente können Oxide des Cers, Zinks, Siliciums, Eisens und Titans bzw. deren Mischungen sein. Besonders bevorzugt ist Titandioxid enthalten. Die Pigmente können in einem Gehalt von 1 bis 20 Gew.-%, vorzugsweise 1 bis 10 Gew.-% oder 2 bis 10 Gew.-% bezogen auf das Gesamtgewicht des Sonnenschutzmittels enthalten sein.

Das Sonnenschutzmittel kann weitere übliche Komponenten enthalten wie Parfüme, Konservierungsstoffe, Füllstoffe, Emulgatoren, Stabilisatoren, Hautpflegemittel, hautberuhigende Stoffe wie Panthenol, Farbstoffe, Silikone, Schäumer, Feuchthaltemittel, Vitamine oder dergleichen.

Besonders bevorzugt liegt das Sonnenschutzmittel in Form einer Emulsion vor, beispielsweise einer Öl-in-Wasser-Emulsion oder ein Wasser-in-Öl-Emulsion, gegebenenfalls auch einer Wasser-in-Öl-in-Wasser-Emulsion oder einer Öl-in-Wasser-in-Öl-Emulsion. Der Wassergehalt des Sonnenschutzmittels liegt jeweils vorzugsweise im Bereich von 10 bis 60 Gew.-% bezogen auf das Gesamtgewicht der Zubereitung, vorzugsweise im Bereich von 20 bis 60 Gew.-% oder 30 bis 55 Gew.-%, vorzugsweise im Bereich von 35 bis 50 Gew.-%, was auch für die Stabilität des Sonnenschutzmittels förderlich sein kann.

Die Ölkomponente des Sonnenschutzmittels kann eine oder mehrere der Bestandteile aus der Gruppe Alkylbenzoat, insbesondere mit bis 25 C-Atomen, bis 20 oder 10 bis 16 C-Atomen der Alkylgruppe, Glycerylfettsäureester, insbesondere mit Fettsäuren mit 8 bis 26 C-Atomen, vorzugsweise 10 bis 20 C-Atomen, beispielsweise Glycerylstearat oder Glyceryloleat, beispielsweise Fettsäureglyceride gesättigter und/oder ungesättigter Fettsäuren, z.B. Kokosfettglyceride, Glyceride, Fettalkohole, insbesondere Fettalkohole mit 8 bis 30 C-Atomen, vorzugsweise 12 bis 24 C-Atomen, beispielsweise Cetearylalkohol, Fettsäureester, insbesondere Ester höherer Fettsäuren mit höheren Fettalkoholen, wobei die Fettsäuren 8 bis 24, vorzugsweise 12 bis 18 C-Atome aufweisen und der Fettalkohol 8 bis 24 C-Atome, vorzugsweise 12 bis 18 C-Atome aufweist, beispielsweise Cetylpalmitat, Glucoside, Glycerin, PEG-Ether, PEG-Ester, Wachse, insbesondere mikrokristalline Wachse und dergleichen enthalten.

Weiterhin können in dem Sonnenschutzmittel enthalten sein Dicarbonsäureester, z.B. von Carbonsäuren mit 2 bis 10 C-Atomen, vorzugsweise 4 bis 6 C-Atomen, insbesondere Adipinsäure, mit C1-C10 Alkoholen, vorzugsweise C2-C4 Alkoholen, insbesondere Butanol, beispielsweise Dibutyladipat, Fettsäureglucoside mit C6-C30 Fettsäuren, vorzugsweise C8-C24, vorzugsweise C12-C18, Laurylglycoside, Siloxynpolymere wie Dimethicone, Methylhexylglycerin, Phenoxyethanol, Tocopherylacetat, ionische, anionische oder neutrale Tenside und dergleichen.

Dicarbonsäureester können in einem Gehalt von 1-25 oder 2-20 Gew.-% bezogen auf das Gesamtgewicht des Sonnenschutzmittels enthalten sein, vorzugsweise 3-15 oder 4-12 Gew.-%, besonders bevorzugt 6-10 Gew.-%-. Alkylbenzoate können in einem Gehalt von 0,5-20 oder 1-10 Gew.-% bezogen auf das Gesamtgewicht des Sonnenschutzmittels enthalten sein, vorzugsweise 1,5-8 oder 2-6 Gew.-%.

Das Sonnenschutzmittel kann insbesondere in Form einer Creme, Lotion, Milch, oder eines Puders, eines Stiftes, eines Sprays oder eines Gels vorliegen.

Die Erfindung sei nachfolgend anhand eines Ausführungsbeispiels beschrieben.

Das erfindungsgemäße Sonnenschutzmittel kann beispielsweise die folgen de Zusammensetzung aufweisen:

| | |
|---|---|
| Aqua | 43,8500 |
| Dibutyl Adipate | 8,0000 |
| Octocrylene | 8,0000 |
| Titanium Dioxide, Dimethicone, Silica | 6,0000 |
| C12-15 Alkyl Benzoate | 4,0000 |
| Glyceryl Stearate, Cetearyl Alcohol, | |
| Cetyl Palmitate, Cocoglycerides (jeweils) | 4,0000 |
| Butyl Methoxydibenzoylmethane | 3,8000 |
| Diethylamino Hydroxybenzoyl Hexyl Benzoate | 3,5000 |
| Glycerin | 3,0000 |
| Lauryl Glucoside, Polyglyceryl-2 Dipolyhydroxy- | |
| stearate | 3,0000 |
| Dimethicone | 2,0000 |
| PVP/Eicosene Copolymer | 2,0000 |
| Microcrystalline Cellulose, Cellulose Gum | 1,5000 |
| Panthenol | 1,3500 |
| Bis-Ethylhexyloxyphenol Methoxyphenyl Triazine | 1,0000 |
| Diethylhexyl Butamido Triazone | 1,0000 |
| Ethylhexylglycerin, Phenoxyethanol | 1,0000 |
| Tocopheryl Acetate | 1,0000 |
| Sodium Cetearyl Sulfate | 0,7500 |
| Benzyl Alcohol, Dehydroacetic Acid (jeweils) | 0,4000 |
| Parfum | 0,3000 |
| Xanthan Gum | 0,2000 |
| Sorbic Acid | 0,1500 |
| Disodium EDTA | 0,1000 |
| Inulin, Lecithin, Phenoxyethanol (jeweils) | 0,1000 |

Der pH-Wert der Zubereitung kann durch organische Säuren auf 3,5 bis 5,5, beispielsweise 4,0 eingestellt sein. Das Sonnenschutzmittel weist einen Lichtschutzfaktor SPF von 50 auf und liegt als Creme vor.

Alternativ kann das UV-Filtersystem des erfindungsgemäßen Sonnenschutzmittels auch in Kombination ein Imidazol, insbesondere Natriumphenylbenzimidazolsulfonat, ein Crylen wie Octocrylen, ein Dibenzoylmethan wie Butylmethoxydibenzoylmethan und ein Triazon wie Diethylhexylbutamidotriazon aufweisen. Beispiele für im erfindungsgemäßen Sonnenschutzmittel einsetzbare Lichtschutzfiltersysteme sind in der folgenden Tabelle angegeben:

| Zubereitung | Licht schutz faktor | Natriumphenyl- Benzimidazolsulfonat | Octocrylen | Butylmethoxydibenzoylmethan | Diethylhexyl-Butamidotriazon |
|---|---|---|---|---|---|
| 1 | Creme/ Lotion SPF 30 | 1,0 | 8,0 | 1,5 | 2,0 |
| 2 | Creme/ Lotion SPF 40 | 2,0 | 8,0 | 1,5 | 2,0 |
| 3 | Creme/ Lotion SPF 50 | 2,0 | 8,0 | | 2,0 |

Die UV-Filter-Komponenten nach obiger Tabelle können in einem erfindungsgemäßen Sonnenschutzmittel mit pH 3,5 bis 5,5 eingesetzt werden. Das Trägersystem kann wie in dem Ausführungsbeispiel berschrieben sein oder dem jeweiligen Anwendungsfall angepasst sein. Das Sonnenschutzmittel weist auch bei niedrigen Temperaturen eine sehr lange Lagerbeständigkeit ohne Neigung zur Auskristallisation auf.

## Patentansprüche

1. Sonnenschutzmittel, enthaltend einen kosmetisch oder dermatologisch akzeptablen Träger und als UV-Filter
(i) mindesten ein Zimtsäureester (Crylen)
(ii) mindestens ein Benzoylmethan und/oder mindestens ein Imidazol und
(iii) mindestens ein Triazin und/oder mindestens ein Triazon,
wobei das Sonnenschutzmittel einen pH-Wert von 3,5 bis 5,5 und einen Lichtschutzfaktor SPF von ≥ 30 aufweist.

2. Sonnenschutzmittel nach Anspruch 1, **dadurch gekennzeichnet, dass** mindestens einer der Stoffe ausgewählt aus der Gruppe Inulin, Dextran, Dextransulfat enthalten ist.

3. Sonnenschutzmittel nach Anspruch 2, **dadurch gekennzeichnet, dass** die Stoffe Inulin, Dextran, Dextransulfat einzeln oder in Kombination in einem Gehalt von ≥ 0,02 Gew.-% enthalten sind.

4. Sonnenschutzmittel nach einem der Ansprüche 1 bis 3,
**dadurch gekennzeichnet, dass** das Crylen Octocrylen ist.

5. Sonnenschutzmittel nach einem der Ansprüche 1 bis 4, **dadurch gekennzeichnet , dass** das Crylen in einem Gehalt von ≥ 2 Gew.-% enthalten ist.

6. Sonnenschutzmittel nach einem der Ansprüche 1 bis 5, **dadurch gekennzeichnet , dass** das Benzoylmethan ein Dibenzoylmethan ist.

7. Sonnenschutzmittel nach einem der Ansprüche 1 bis 5, **dadurch gekennzeichnet, dass** das Benzoylmethan in einem Gehalt von ≥ 0,2 Gew.-% enthalten ist.

8. Sonnenschutzmittel nach einem der Ansprüche 1 bis 7, **dadurch gekennzeichnet, dass** das Triazin ein Phenolalkoxyphenyltriazin ist.

9. Sonnenschutzmittel nach einem der Ansprüche 1 bis 8, **dadurchgekennzeichnet**, **dass** das Triazin in einem Gehalt von ≥ 0,1 Gew.-% enthalten ist.

10. Sonnenschutzmittel nach einem der Ansprüche 1 bis 9, **dadurch gekennzeichnet, dass** das Triazon ein Amidotriazon ist.

11. Sonnenschutzmittel nach einem der Ansprüche 1 bis 10, **dadurch gekennzeichnet , dass** das Triazon in einem Gehalt von ≥ 0,1 Gew.-% enthalten ist.

12. Sonnenschutzmittel nach einem der Ansprüche 1 bis 11, **dadurch gekennzeichnet, dass** das Imidazol ein Phenylbenzylimidazolsalz ist.

13. Sonnenschutzmittel nach einem der Ansprüche 1 bis 12, **dadurch gekennzeichnet, dass** das Imidazol in einem Gehalt von ≥ 0,1 Gew.-% enthalten ist.

14. Sonnenschutzmittel nach einem der Ansprüche 1 bis 13, **dadurch gekennzeichnet, dass** der pH-Wert 4,5 bis 5,5 beträgt.

15. Sonnenschutzmittel nach einem der Ansprüche 1 bis 14, **dadurch gekennzeichnet, dass** Lecithin enthalten ist.

16. Sonnenschutzmittel nach einem der Ansprüche 1 bis 15, **dadurch gekennzeichnet, dass** das Sonnenschutzmittel in Form einer Emulsion mit einem Wassergehalt im Bereich von 10 bis 60 Gew.-% bezogen auf das Gesamtgewicht der Zubereitung vorliegt.
